# EUROPEAN PATENT APPLICATION

(11) **EP 4 843 547 A1**
(43) Date of publication of application: **25.11.2026**
(21) Application number: 25220237.9
(22) Date of filing: 02.12.2025
(51) Int. Cl.: A61F 13/15, A61F 13/494, A61F 13/513, A61F 13/511

(54) **ABSORBENT ARTICLE WITH NOVEL BARRIER CUFFS**

(30) Priority: 19.05.2025 EP 25177327
(71) Applicant: Ontex BV, 9255 Buggenhout (BE); Ontex Group NV, 9320 Erembodegem (BE)
(72) Inventor: BUYSSE, Michiel, 9968 Oosteeklo (BE); PSCHYKLENK, Lukas, 56761 Müllenbach (DE); FRANSEN, Markus, 56269 Dierdorf (DE)
(74) Representative: Macchetta, Andrea

(57) **Abstract**

An absorbent article, such as a diaper, (diaper) pant or other similar disposable product, includes a substantially liquid permeable topsheet, a liquid impermeable backsheet, and an absorbent core. A pair of barrier leg cuffs are formed, at least in part, by a zone-treated topsheet. The topsheet has a central hydrophilic section for fluid acquisition and hydrophobic lateral regions that extend to form at least an inner facing layer of the barrier leg cuffs, improving leakage prevention. The hydrophobic regions may be joined to a further distinct outer facing layer with elastic elements, or may comprise an inner folded edge with encapsulated elastics.

## Description

### TECHNICAL FIELD

The present disclosure pertains to the technical field of absorbent hygiene articles such as disposable diapers or (diaper) pants, training pants, disposable incontinence diapers or pants, pads, provided with barrier cuffs reducing the risk of side leakage and offering comfort.

### BACKGROUND

Absorbent articles such as diapers, training pants, and incontinence products are designed to absorb and contain body fluids and waste. These articles typically include a topsheet that contacts the wearer's skin, a liquid-impermeable backsheet to prevent leakage, and an absorbent core between the topsheet and backsheet to absorb and retain fluids. To further prevent leakage, many absorbent articles incorporate barrier leg cuffs that extend upwardly from the inner surface of the article along the longitudinal edges.

A common approach in the prior art is to construct these barrier leg cuffs from separate pieces of material that are then bonded to the topsheet at the so-called "cuff feet" area. This bonding step, however, can introduce several drawbacks. First, the adhesives used for bonding can be stiff and uncomfortable for the wearer. Second, the bonding process itself can create a potential leakage path, especially if the bond is not complete or degrades over time. Third, the bonding may be negatively influenced by migration of the spin finish typically used to treat the topsheet to make it hydrophilic.

Another issue in traditional absorbent articles is managing fluid distribution. It is desirable to have a topsheet that quickly acquires fluid and directs it to the absorbent core. This is typically achieved by treating the topsheet with a hydrophilic spin finish. However, if the entire topsheet is hydrophilic, fluid can spread laterally and potentially leak out of the sides of the article.

Therefore, a problem exists in the art to provide an absorbent article that reduces or eliminates leakage, particularly in the crotch area, while maintaining wearer comfort and effective fluid management.

### SUMMARY

In a first aspect, the present disclosure relates to an absorbent article comprising a topsheet; a liquid impermeable backsheet; an absorbent core disposed at least partially between the topsheet and the backsheet; and a pair of barrier leg cuffs, each barrier leg cuff comprising an inner cuff element; and characterised in that the topsheet forms at least a portion of each of said barrier leg cuffs; and in that the topsheet is zone treated so that a central section of the topsheet is hydrophilic and is interposed between at least two hydrophobic lateral regions that are oppositely disposed relative to a longitudinal centerline of the absorbent article; and preferably wherein the hydrophobic lateral regions of the topsheet form at least an inner facing layer of the barrier leg cuffs. This configuration may provide improved leakage protection by utilizing the topsheet material to form the cuffs and strategically controlling the surface properties of the topsheet.

In a second aspect, the present disclosure relates to a method or the manufacture of an absorbent article, said article preferably according to any of the embodiments herein described. The method comprises the steps of providing a topsheet; zone treating the topsheet to form a central hydrophilic section and at least two hydrophobic lateral regions oppositely disposed relative to a longitudinal centerline of the absorbent article; providing a liquid impermeable backsheet, and an absorbent core; positioning the absorbent core at least partially between the topsheet and the backsheet; extending the topsheet beyond longitudinal edges of the absorbent core; folding the extended portions of the topsheet comprising the hydrophobic lateral regions to form at least an inner facing layer of a pair of barrier leg cuffs; and optionally securing the hydrophobic lateral regions of the topsheet to a further distinct outer facing layer to form a pair of barrier leg cuffs. This method may allow for efficient and cost-effective production of an absorbent article with enhanced leakage protection and fluid management.

According to a preferred embodiment of the invention, an absorbent the hydrophobic region of the topsheet is joined to a further distinct outer facing layer of the barrier leg cuffs with one or more elastic elements being sandwiched therebetween. This enhances the fit and containment of the cuffs by providing elasticity and a secure seal against the wearer's skin.

According to a preferred embodiment of the invention, the hydrophobic region of the topsheet comprises an inner folded edge with one or more elastics encapsulated therein to form each of the pair of barrier leg cuffs, and preferably wherein said cuffs are free of a further distinct layer. This provides simplified cuff construction with enhanced comfort and reduced risk of irritation.

According to a preferred embodiment of the invention, the topsheet is a hydrophobic nonwoven that is treated with a hydrophilic spin finish within said central section such that the hydrophobic lateral regions are substantially free of said hydrophilic spin finish. This enables precise control over fluid acquisition and distribution within the absorbent article for desirable functionality as will be described in more detail herein.

According to a preferred embodiment of the invention, the topsheet is a nonwoven layer selected from the group consisting of: spunbond, meltblown, carded, and combinations thereof. This provides flexibility in material selection based on desired performance characteristics.

According to a preferred embodiment of the invention, each barrier leg cuffs is an upstanding barrier leg cuff and comprise a single layer of nonwoven web. This simplifies the manufacturing process while maintaining effective leakage protection.

According to a preferred embodiment of the invention, each barrier leg cuffs is an upstanding barrier leg cuff and comprise at least two layers of nonwoven web, and preferably wherein the majority of said cuff is free of adhesive or other joining means between said two layers so that an air gap may be formed between them. This creates an even more resistant protection against leakage.

According to a preferred embodiment of the invention, the distinct outer facing layer is selected from a material that is the same or different to that of the topsheet, and preferably wherein said material is selected from the group consisting of nonwoven, film or combinations thereof. This allows for customization of the cuff properties based on the selection of the outer facing layer material.

According to a preferred embodiment of the invention, the material of the distinct outer facing layer is a nonwoven selected from the group consisting of spunbond, meltblown, and combinations thereof. This provides a cost-effective and performant outer facing layer.

According to a preferred embodiment of the invention, further comprises an outer cover that is associated with the backsheet, preferably at a garment facing surface thereof; more preferably wherein the outer cover is a hydrophobic nonwoven layer. This provides enhanced containment and a garment-facing surface that is resistant to liquid penetration as well as allowing to provide greater softness and reduced skin irritation.

According to a preferred embodiment of the invention, the distinct outer facing layer is part of the liquid impermeable backsheet of the absorbent article; and preferably wherein the backsheet is a film. This simplifies the construction and reduces material costs.

According to a preferred embodiment of the invention, the distinct outer facing layer is part of the outer cover of the absorbent article. This further integrates the cuff structure with the outer layers of the article and may provide a softer skin-contact that helps avoid skin irritation.

According to a preferred embodiment of the invention, the outer cover is sized to be larger than the backsheet, preferably such that it may extend beyond the backsheet to exclusively form the distinct outer facing layer. This creates a seamless transition between the outer cover and the cuff for added comfort and reduced formation of wrinkles at the article edges.

According to a preferred embodiment of the invention, each of the cuffs comprise a cutaneous sealing strut and an upstanding connecting strut adjoining said cutaneous sealing strut, preferably wherein each of the cuffs is substantially T-shaped. This improves the sealing performance of the cuffs against the wearer's skin.

According to a preferred embodiment of the invention, the cutaneous sealing strut has a first longest dimension, and the upstanding connecting strut has a second longest dimension; and wherein a ratio of first longest dimension/second longest dimension is from 0.8 to 2.5, preferably from 0.9 to 2.2, even more preferably between 1 and 2; preferably wherein the first longest dimension runs parallel to a width of a skin-contact surface and the second longest dimension runs substantially perpendicular thereto. This optimizes the cuff geometry for effective sealing and comfort.

### BRIEF DESCRIPTION OF THE FIGURES

The disclosure can be better understood with reference to the following drawings and description. The components in the figures are not necessarily to scale, emphasis instead being placed upon illustrating various preferred embodiments of the disclosure. These embodiments are provided as non-limiting examples to aid in understanding the inventive concepts.
**Fig. 1** Top plan view of an absorbent article illustrating a general configuration according to an aspect of the disclosure. The absorbent article may be a diaper, an incontinence brief, or another similar absorbent product. The view shows the arrangement of the topsheet, backsheet, absorbent core, and barrier leg cuffs in one possible configuration.
**Fig. 2** Section view of an absorbent article according to Fig. 1 about section A-A. This cross-sectional view provides a more detailed look at the layers and components of the absorbent article along the indicated section line. It illustrates a possible configuration of the topsheet, backsheet, absorbent core, and barrier leg cuffs, and elastic elements.
**Fig. 3** Section view identical to Fig.2 but with fewer components being called out by reference numerals to improve intelligibility.
**Fig. 4** A and B show exemplary steps of forming cuffs by joining and folding the topsheet to further distinct outer facing layers according to an embodiment herein.
**Fig. 5A** Section view of a T-shaped cuff according to an embodiment of the disclosure.
**Fig. 6A-C** Absorbent articles according to embodiments of the present disclosure comprising channels. These figures illustrate possible embodiments of the absorbent article incorporating channels within the absorbent core structure. The channels may vary in shape, size, and arrangement, and the figures provide non-limiting examples of such variations.
**FIG. 7** is a top plan view of a topsheet comprising zone treatment according to embodiments herein.

### DETAILED DESCRIPTION

Unless otherwise defined, all terms used in disclosing characteristics of the disclosure, including technical and scientific terms, have the meaning as commonly understood by one of ordinary skill in the art to which this disclosure belongs. By means of further guidance, term definitions are included to better appreciate the teaching of the present disclosure.

As used herein, the following terms have the following meanings:
"A", "an", and "the" as used herein refers to both singular and plural unless the context clearly dictates otherwise. By way of example, "a compartment" refers to one or more compartments.

"About" as used herein referring to a measurable value such as a parameter, an amount, a temporal duration, and the like, encompass variations of +/-20% or less, preferably +/-10% or less, more preferably +/-5% or less, even more preferably +/-1% or less, and still more preferably +/-0.1% or less of and from the specified value, in so far such variations are appropriate to perform in the disclosed invention. However, it is to be understood that the value to which the modifier "about" refers is itself also specifically disclosed.

"Comprise", "comprising", "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

The recitation of numerical ranges by endpoints includes all numbers and fractions subsumed within that range, as well as the recited endpoints.

"Absorbent article" refers to devices that absorb and contain liquid, and more specifically, refers to devices that are placed against or in proximity to the body of the wearer to absorb and contain the various exudates discharged from the body. Absorbent articles include but are not limited to diapers, adult incontinence briefs, training pants and diaper holders and liners, Absorbent articles preferably comprise a longitudinal axis and a transversal axis perpendicular to said longitudinal axis. The longitudinal axis is hereby conventionally chosen in the front-to-back direction of the article when referring to the article being worn, and the transversal axis is conventionally chosen in the left-to-right direction of the article when referring to the article being worn. The absorbent article includes a chassis. The absorbent article may include liquid wicking layers, liquid intake layers, liquid distribution layers, transfer layers, barrier layers, wrapping layers and the like, as well as combinations thereof. Disposable absorbent articles and the components thereof can operate to provide a skin-facing surface and a garment-facing surface.

A "chassis" of a disposable absorbent articles can include a liquid pervious topsheet, a backsheet joined to the top sheet, and an absorbent core positioned and held between the top sheet and the back sheet. The top sheet is operatively permeable to the liquids that are intended to be held or stored by the absorbent article, and the back sheet may or may not be substantially impervious or otherwise operatively impermeable to the intended liquids. The chassis generally comprises a front (F) waist portion or region, a back (B) waist portion or region, an intermediate crotch portion or region which interconnects the front and back waist portions. When used herein, reference to a "front" portion refers to that part of the chassis which is generally located on the front of a wearer when in use. Reference to the "back" portion refers to the portion of the chassis generally located at the back of the wearer when in use, and reference to the "crotch" portion refers to that portion which is generally located between the legs of a wearer when in use. The crotch portion is an area where repeated fluid surge typically occurs.

The "absorbent core" is the absorbent structure disposed between the topsheet and the backsheet of the chassis and is capable of absorbing and retaining liquid body exudates. The size and the absorbent capacity of the absorbent core should be compatible with the size of the intended wearer and the liquid loading imparted by the intended use of the chassis. Further, the size and the absorbent capacity of the absorbent core can be varied to accommodate wearers ranging from infants through adults. It may be manufactured in a wide variety of shapes (for example, rectangular, trapezoidal, T-shape, I-shape, hourglass shape, etc.) and from a wide variety of materials. Examples of commonly occurring absorbent materials are cellulosic fluff pulp, tissue layers, highly absorbent polymers (so called superabsorbent polymer particles (SAP)), absorbent foam materials, absorbent nonwoven materials or the like. It is common to combine cellulosic fluff pulp with superabsorbent polymers in an absorbent core. It can contain embossed channels, channels substantially free of material, channels with lower basis weight than the rest of the core, etc. The absorbent core is disposed between the topsheet and the backsheet; both the topsheet and the backsheet generally extend beyond the side edges of the absorbent core. The juxtaposed areas of the topsheet and the backsheet are typically secured together such as by adhesive.

"Acquisition and distribution layer", "ADL" or "surge management portion" refers to a sublayer which preferably is a nonwoven wicking layer under the top sheet of an absorbent product, which speeds up the transport and improves distribution of fluids throughout the absorbent core. The surge management portion is typically less hydrophilic than the retention portion, and has the ability to quickly collect and temporarily hold liquid surges, and to transport the liquid from its initial entrance point to other parts of the absorbent structure, particularly the retention portion. This configuration can help to prevent the liquid from pooling and collecting on the portion of the absorbent garment positioned against the wearer's skin, thereby reducing the feeling of wetness by the wearer. Preferably, the surge management portion is positioned between the top sheet and the retention portion.

The term "adhesive" as used herein is intended to refer to any suitable hot melt, water or solvent borne adhesive that can be applied to a surface in the required pattern or network of adhesive areas. Accordingly, suitable adhesives include conventional hot melt adhesives, pressure-sensitive adhesives and reactive adhesives (i.e., polyurethane).

"Bonded" refers to the joining, adhering, connecting, attaching, or the like, of at least two elements. Two elements will be considered to be bonded together when they are bonded directly to one another or indirectly to one another, such as when each is directly bonded to intermediate elements. "Join", "joining", "joined", or variations thereof and "attach", "attaching", "attached", or variations thereof, when used in describing the relationship between two or more elements, means that the elements can be connected together in any suitable manner, such as by heat sealing, ultrasonic bonding, thermal bonding, by adhesives, stitching, or the like. Further, the elements can be joined or attached together either directly or indirectly, i.e. they may have one or more elements interposed between them, all of which are connected together. For the sake of clarity, adhesive is not to be considered as an intervening element for the definition of direct or indirect attachment. When describing an attachment "without intervening layer of material" or "without any intervening material", this does not exclude the presence of adhesive.

As used herein, the term "adhesive bonding" means a bonding process which forms a bond by application of an adhesive. Such application of adhesive may be by various processes such as slot coating, spray coating and other topical applications. Further, such adhesive may be applied within a product component and then exposed to pressure such that contact of a second product component with the adhesive containing product component forms an adhesive bond between the two components.

As used therein, the term "associated" encompasses configurations in which two elements are directly or indirectly joined. For example configurations where top sheet is directly joined to back sheet by affixing top sheet directly to back sheet, and configurations wherein top sheet is joined to back sheet by affixing top sheet to intermediate members which in turn are affixed to back sheet. Top sheet and back sheet can be affixed directly to each other by attachment means such as an adhesive, sonic bonds, thermal bonds or any other attachment means known in the art. For example, a uniform continuous layer of adhesive, a patterned layer of adhesive, a sprayed pattern of adhesive or an array of separate lines, swirls or spots of construction adhesive may be used to affix top sheet to back sheet. It should be readily appreciated that the above-described attachment means may also be employed to interconnect and assemble together the various other component parts of the article described herein.

The term "backsheet" refers to a material forming a liquid impervious cover of the chassis of the absorbent article. The back sheet prevents the exudates contained in the absorbent core from wetting articles such as bedsheets and overgarments which contact the disposable absorbent article. The back sheet may be a composite layer composed of multiple components assembled side-by-side or laminated. The back sheet may be the same or different in different parts of the chassis. At least in the area of the absorbent core, the back sheet comprises a liquid impervious material in the form of a thin plastic film, e.g., a polyethylene or polypropylene film, a nonwoven material coated with a liquid impervious material, a hydrophobic nonwoven material, which resists liquid penetration, or a laminate of a plastic film and a nonwoven material. The back sheet material may be breathable so as to allow vapour to escape from the absorbent material, while still preventing liquids from passing there through. Examples of breathable back sheet materials are porous polymeric films, nonwoven laminates of spunbond and meltblown layers and laminates of porous polymeric films and nonwoven materials, in such laminates, the polymeric film can have the same or a different width than the nonwoven material. The term "breathable" refers to films having a water vapor transmission rate (WVTR) of at least 300 grams/m² - 24 hours.

As used herein, the "skin-facing" or "bodyside" or " body-facing" or "user facing" or "inner" side or surface means that surface of the article or component which is intended to be disposed toward or placed adjacent to the body of the wearer during ordinary use, while the "outward", "outward-facing", "garment-facing" or "garment" or "outer" side or surface is on the opposite side, and is intended to be disposed to face away from the wearer's body during ordinary use. Such outward surface may be arranged to face toward or placed adjacent to the wearer's undergarments when the absorbent article is worn.

As used herein, the terms "elastic", "elastomeric", "elasticity" or derivations thereof are used to describe the ability of various materials and objects comprised of such to reversibly undergo deformation under stress, e.g., become stretched or extended, in at least one direction when a force is applied to the material and to resume substantially to their original dimensions upon relaxing, i.e., when the force is released, without rupture or breakage. Preferably, it refers to a material or composite which can be elongated in at least one direction by at least 50% of its relaxed length, i.e., elongated to at least 150% of its relaxed length, and which will recover upon release of the applied tension at least 40% of its elongation. Accordingly, upon release of the applied tension at 50% elongation, the material or composite contracts to a relaxed length of not more than 130% of its original length. Examples of suitable elastomer materials include polyether-polyamide block copolymers, polyurethanes, synthetic linear A-B-A and A-B block copolymers, chlorinated rubber/EVA (ethylene-vinyl acetate) blends, EPDM (ethylene-propylene diene monomer) rubbers, EPM (ethylene-propylene monomer) rubbers, blends of EPDM/EPM/EVA, and the like.

As used herein, the term "impermeable" or "impervious" generally refers to articles, chassis and/or elements that are substantially not penetrated by aqueous fluid through the entire thickness thereof under a pressure of 1.0 kPa or less. Preferably, the impermeable article or element is not penetrated by aqueous fluid under pressures of 3.4 kPa or less. More preferably, the impermeable article or element is not penetrated by fluid under pressures of 6.8 kPa or less. An article or element that is not impermeable is permeable.

The term "flat configuration" or "laid-flat state" or "fully stretched state" or "extended state" or "flat-out" configuration is intended to refer to the article when it is flattened into a plane or is substantially flattened into a plane and is used in contrast to when the article otherwise positioned, such as when the article is folded or shaped in or for use by a wearer.

The use of the term "layer" can refer, but is not limited, to any type of substrate, such as a woven web, nonwoven web, films, laminates, composites, elastomeric materials, or the like, or even formulations, such as adhesives, that form a substrate upon a change in conditions (e.g. solidification of a hotmelt adhesive when the temperature drops below a predetermined amount). A layer can be liquid and air permeable, permeable to air but impermeable to liquids, impermeable both to air and liquid, or the like. When used in the singular, it can have the dual meaning of a single element or a plurality of elements.

The term "topsheet" refers to a liquid permeable material sheet forming the inner cover of the chassis of an absorbent article and which in use is placed in direct contact with the skin of the wearer. The topsheet is typically employed to help isolate the wearer's skin from liquids held in the absorbent structure. The top sheet can comprise a nonwoven material, e.g., spunbond, meltblown, carded, hydroentangled, wetlaid or any other type of nonwoven. Suitable nonwoven materials can be composed of man-made fibres, such as polyester, polyethylene, polypropylene, viscose, rayon etc. or natural fibers, such as wood pulp or cotton fibres, hemp, and the like, or other natural or naturally-derived fibers, or from a mixture of two or more natural or man-made fibres. The top sheet material may further be composed of two fibres, which may be bonded to each other in a bonding pattern. Further examples of top sheet materials are porous foams, apertured plastic films, laminates of nonwoven materials and apertured plastic films etc. The materials suited as top sheet materials should be soft and non-irritating to the skin and be readily penetrated by body fluid, e.g., urine or menstrual fluid. The inner coversheet may further be different in different parts of the chassis of an absorbent article. The top sheet fabrics may be composed of a substantially hydrophobic material, and the hydrophobic material may optionally be treated with a surfactant or otherwise processed to impart a desired level of wettability and hydrophilicity.

The term "nonwoven" or "nonwoven fabric or web" means a sheet material having a structure of individual fibers or threads which are interlaid, but not in a regular manner such as occurs with knitting or weaving processes. Nonwoven fabrics or webs have been formed from many processes such as for example, meltblowing processes, spunbonding processes, and bonded carded web processes. Nonwovens may be embossed via an embossing process whereby a pattern is pressed into the fabric, usually by passing the material between rolls with little clearance, and where one or both rolls has a raised design. "Three dimensional embossed nonwovens", also called "embossed nonwovens" may provide enhanced softness and cushioning.

As used herein, an "air-through-bonded" nonwoven, is a nonwoven structure primarily formed by a process that comprises the application of heated air to the surface of the nonwoven fabric. During the through air bonding process, heated air flows through holes in a plenum above the nonwoven material. Unlike hot ovens, which push air through the material, the through air process uses negative pressure of suction to pull the air through an open conveyor apron holding nonwoven as it is drawn through the oven. Pulling air through the material allows the rapid and even transmission of heat to minimize distortion of the nonwoven material. The binding agents used in the through air bonding process include crystalline binder fibers and powders, which melt to form molten droplets throughout the cross-section of the nonwoven. As the material is cooled, bonding occurs at these droplet points.

As used herein, the term "transverse" or "lateral" refers to a line, axis, or direction which lies within the plane of the absorbent article and is generally perpendicular to the longitudinal direction.

Embodiments according to the disclosure will now be described. It is understood that technical features described in one or more embodiments may be combined with one or more other embodiments without departing from the intention of the disclosure and without generalization therefrom.

### THE ABSORBENT ARTICLE

The present disclosure pertains to embodiments of an absorbent article (1), including but not limited to disposable diapers, training pants, adult incontinence products, and feminine hygiene products, designed for the absorption and containment of bodily fluids. Most preferably, the absorbent article herein is a disposable pant. These embodiments aim to improve leakage prevention while maintaining wearer comfort and fit at a lowest possible cost.

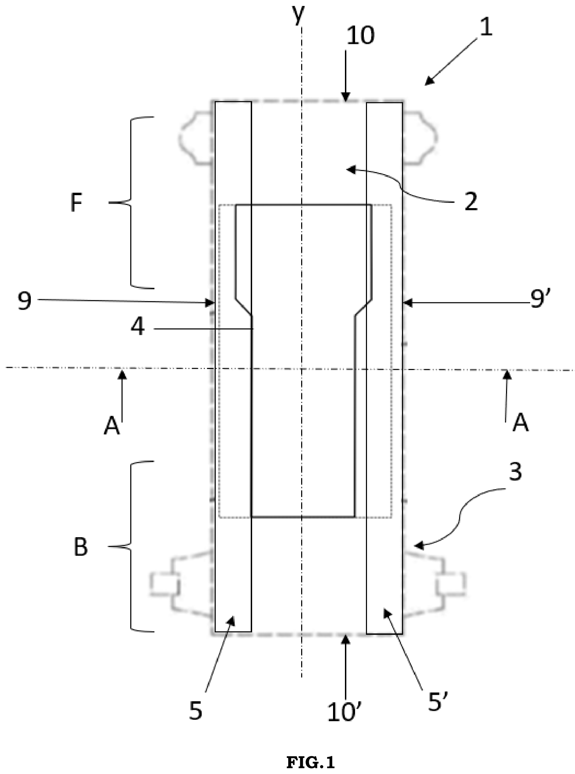

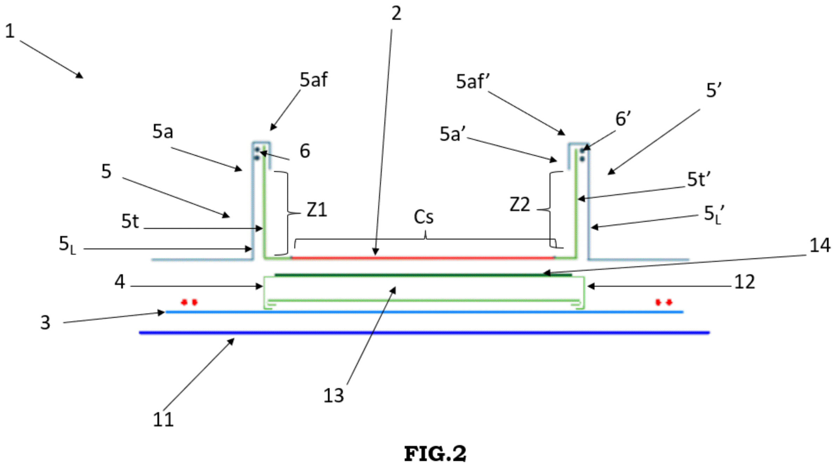

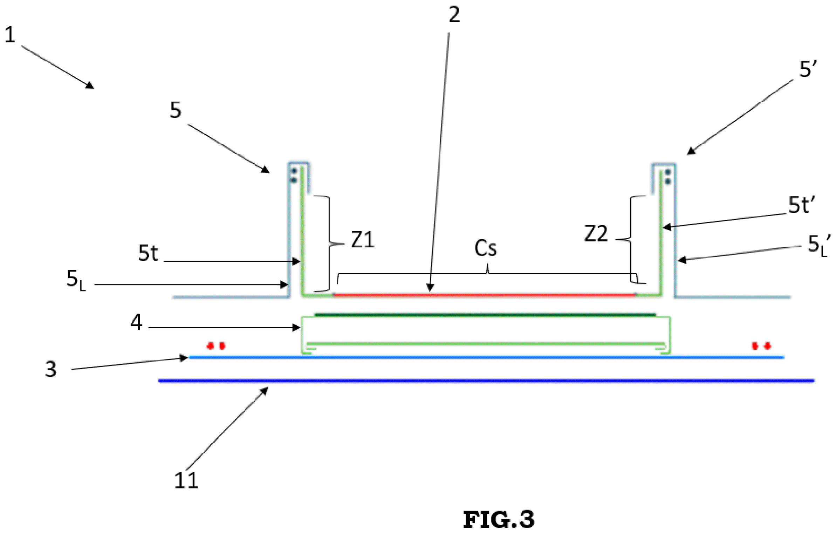

As for example illustrated in Fig.1-3, absorbent articles herein comprise: a liquid permeable topsheet (2), a liquid impermeable backsheet (3), and an absorbent core (4) disposed at least partially between the topsheet (2) and the backsheet (3). A pair of barrier leg cuffs (5, 5') are included, each comprising an inner cuff element (5a, 5a'). The topsheet (2) is generally liquid permeable across at least a central section thereof to facilitate rapid fluid acquisition, while the liquid impermeable backsheet (3) prevents fluid leakage. The barrier leg cuffs (5,5') are positioned to provide enhanced containment and further prevent leakage from the absorbent article.

Topsheets (2) herein form at least a portion of each of the barrier leg cuffs (5, 5'), and further are zone treated so that a central section of the topsheet (2) is hydrophilic and is interposed between at least two hydrophobic lateral regions (Z1, Z2) that are oppositely disposed relative to a longitudinal centerline of the absorbent article. While the topsheet (2) is typically liquid permeable, it is not essential that it be permeable throughout its entire surface. Indeed, the hydrophobic zones (Z1, Z2) of the topsheet will preferably be liquid impermeable in order to enhance their barrier function. This unique combination provides a continuous material extending from the topsheet into the cuffs, reducing potential leak paths, and strategically managing fluid distribution through the zone treatment.

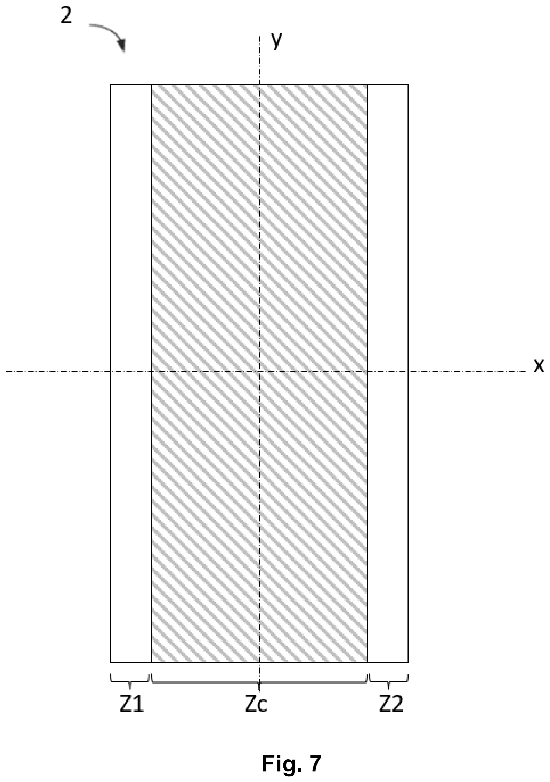

As exemplarily illustrated in Fig.7, the topsheet (2) herein may comprise one or more first zones (Zc) [preferably corresponding to the central section (Cs) of the topsheet (2) as described herein] and at least two second zones (Z1, Z2) wherein the first zone(s) (Zc) is substantially hydrophilic [typically meaning that at least semi-durable hydrophilic properties are imparted as explained in more detail in embodiments herein] and wherein the second zones (Z1, Z2) are hydrophobic; and preferably attached portion(s) of each of said further distinct outer facing layer (5_{L}, 5_{L}') of the barrier leg cuffs (5, 5'), when present, overlap at least one of the second zones (Z1, Z2), and does not substantially overlap [typically meaning that minor overlaps, e.g. due to production tolerances, are encompassed], preferably does not overlap, the first zone(s) (Z1); and/or wherein the attached portion(s) of each of said distinct outer facing layer (5_{L}, 5_{L}') is positioned proximal or adjacent to a second zone (Z1, Z2) and distal from said first zone(s) (Zc) such that the attached portion (23) is separated from said first zone(s) (Z1) by said second zone (Z2) preferably in a width direction extending substantially parallel to the lateral axis X-X. Advantageously this arrangement allows to significantly reduce the risk of leakage that could otherwise arise from liquid permeating through the hydrophilic portions of the topsheet and under elements forming the cuffs.

The absorbent article (1) is generally constructed around a central chassis, which provides a structural foundation and functional elements for fluid management. The chassis comprises the liquid permeable topsheet (2), the liquid impermeable backsheet (3), and the absorbent core (4).

To provide a well-defined and reliable containment structure, the pair of barrier leg cuffs (5, 5') are preferably strategically positioned at, or proximal to, a lateral edge (9, 9') of the absorbent article (1). This lateral edge (9, 9') extends substantially parallel to the longitudinal axis (y) of the absorbent article (1), providing a continuous barrier along the sides of the product. The lateral edges (9, 9'), in conjunction with a pair of oppositely disposed transversal edges (10, 10') extending substantially perpendicular to the longitudinal axis (y), generally form a perimeter of the chassis of said absorbent article (1).

Further one or more leg elastics are preferably disposed directly between each of the pair of barrier leg cuffs (5, 5') and at least one of the topsheet (2) and the backsheet (3) generally to provide a gasketing effect, ensuring a snug fit around the wearer's legs and further preventing leakage. By disposing these further elastic elements between the barrier leg cuffs (5, 5') and the topsheet (2) and/or the backsheet (3), the design ensures maximum contact and conformability to the wearer's body in a cost-effective manner. These leg elastics are generally positioned along the longitudinal edges (9, 9') of the chassis and may extend along only a part of the length of the barrier leg cuffs herein.

It is preferred herein that the one or more leg elastics are disposed directly between each of the pair of barrier leg cuffs (5, 5') and the backsheet (3). Securing the leg elastics (8, 8') directly between the barrier leg cuffs (5, 5') and the backsheet (3), may provide several key advantages. First, the lamination strength and overall mechanical integrity of the assembly may be enhanced due to the strong adhesion between the elastics and the film of the backsheet. This configuration offers improved resistance to tearing or delamination, ensuring the leg cuffs maintain their intended position and function throughout the use of the absorbent article (1). Second, this direct securement to the backsheet (3) may ensure that the liquid impermeable film area is positioned as closely as possible to the wearer's skin, indirectly urging the topsheet into closer contact with the skin as well. This intimate contact between the topsheet, backsheet, and skin creates a more effective seal, maximizing the capture and containment of bodily fluids and providing an extra layer of leakage protection. The high securement forces may also ensure that the leg cuffs function as intended throughout the wearing time. For the avoidance of any doubt, the term "directly" in relation to the positioning of the leg elastics, it is understood that the phrase "disposed directly between" is not intended to exclude the presence of adhesive used to secure the leg elastics in their designated location. While the leg elastics are preferably positioned in direct contact with the topsheet (2) and/or backsheet (3), adhesive or other bonding agents may be applied to maintain their position and functionality. The term "directly" is therefore used to indicate that there are no substantial intervening layers of material between the leg elastics and the topsheet (2) and/or backsheet (3), other than suitable amounts of adhesive (or functionally similar bonding means alternative) necessary for securement.

The topsheet (2) generally forms the innermost layer, designed for direct contact with the wearer's skin. Its primary function is to rapidly acquire discharged fluids and transport them to the underlying absorbent core (4), while minimizing rewetting and maintaining a dry and comfortable surface against the skin. To achieve this, the topsheet (2) is generally selected to exhibit softness. Liquid permeability may be achieved through various material properties and structural features, such as pore size and distribution designed for efficient fluid intake, or through capillary action, drawing fluid away from the surface. Surface treatments, in the form of hydrophilic finishes or surfactants, may also be applied to the topsheet (2) to enhance its wettability and liquid acquisition rate. One preferred approach is the use of a zone-treated topsheet with distinct regions of differing wettability. The topsheet (2) may be a nonwoven fabric (e.g., spunbond, meltblown, carded, hydroentangled), an apertured film, or a laminate. Nonwoven fabrics may be composed of synthetic fibers (e.g., polypropylene, polyethylene, polyester), natural fibers (e.g., cotton, cellulose), or combinations thereof. For example, the topsheet (2) may comprise a spunbond nonwoven (e.g. from PFN, 100 Wt% PP).

In a preferred embodiment, the absorbent article comprises a zone-treated topsheet (2) having distinct hydrophilic and hydrophobic regions to optimize fluid management and leakage prevention. Specifically, a central hydrophilic section is interposed between two hydrophobic lateral regions (Z1, Z2) positioned proximal and/or adjacent to the cuffs (5, 5'). This configuration addresses a specific challenge often observed with certain topsheet materials, such as those exhibiting enhanced loft, softness, or premium fluid acquisition, which can exhibit an increased propensity for lateral fluid migration. This migration can occur via capillary action within the topsheet structure, guiding fluid underneath the cuff attachment and towards the article's periphery, leading to leakage. By strategically incorporating hydrophobic zones adjacent to the cuff attachment areas, a further barrier is effectively created that impedes this lateral fluid transport, mitigating the risk of side leakage.

The zone treatment of the topsheet (2) can be achieved through various methods. One method involves the selective application of a hydrophilic spin finish, where a hydrophilic spin finish is applied to the central section of the topsheet (2), while the hydrophobic lateral regions (Z1, Z2) are substantially free of this finish. The hydrophilic finish may be applied by spraying, printing, or other suitable methods, ensuring a controlled and uniform distribution within the central section. Examples of suitable hydrophilic spin finishes include surfactants such as ethoxylated alcohols, ethoxylated esters, and polyethylene glycol. Another suitable method, though less preferred, is the application of a hydrophobic coating, where a hydrophobic coating is applied to the lateral regions (Z1, Z2) of the topsheet (2), rendering them liquid-repellent. The hydrophobic coating may be a polymer, wax, or silicone-based material applied by spraying, printing, or dipping. Alternatively, the lateral regions (Z1, Z2) of the topsheet (2) may be constructed from hydrophobic fibers, while the central section is constructed from hydrophilic fibers. This can be achieved by selectively blending different fiber types during nonwoven fabric formation.

The dimensions and ratios of the zone treatment may be managed for optimized performance. The width of the central hydrophilic section is preferably sufficient to quickly acquire and distribute fluid to the absorbent core (4), while the width of the hydrophobic lateral regions (Z1, Z2) is sufficient to prevent lateral fluid migration and leakage. The ratio of the width of the central hydrophilic section to the width of each hydrophobic lateral region (Z1 or Z2) is preferably between 0.5:1 and 5:1, more preferably between 1:1 and 3:1. For each of the hydrophobic lateral regions (Z1 and Z2), the width may range from 20 mm to 100mm, preferably from 30 mm to 90 mm, even more preferably from 35 mm to 80 mm, and even more preferably from 40 mm to 70 mm, to provide adequate barrier function without compromising comfort. The hydrophilic spin finish add-on in the central section may range from 0.1% to 1.0% by weight of the topsheet (2), providing a balance between wettability and cost-effectiveness.

The backsheet (3) generally forms the outermost layer, preventing leakage of fluids from the absorbent core (4) and protecting clothing and bedding. Backsheets herein are preferably in the form of a film. Key characteristics of the backsheet (3) include liquid impermeability and, optionally, breathability. The liquid impervious film layer may be made of polyethylene (PE). In some embodiments, the backsheet (3) is breathable, allowing water vapor to escape from the absorbent core (4) while maintaining liquid impermeability. A nonwoven outer cover layer (10) may overlay the backsheet (3) and forms the outer surface of the absorbent article (1). The nonwoven outer cover layer may be a single layer or multi-layer structure.

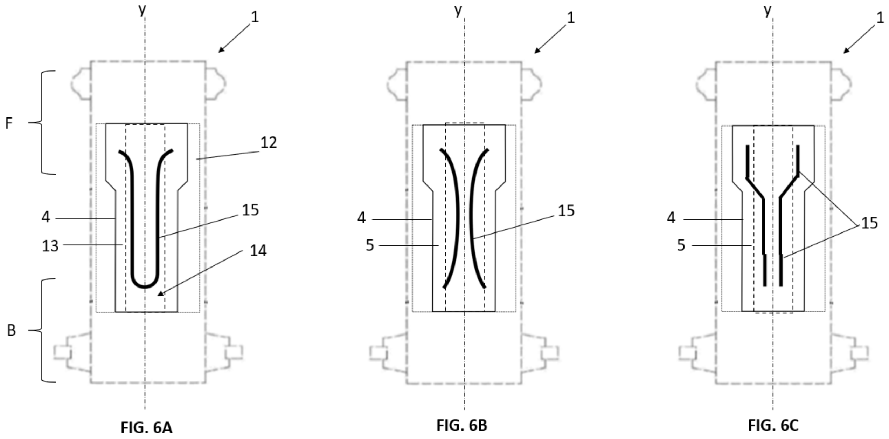

The absorbent core (4) is generally positioned between the topsheet (2) and backsheet (3) and is the primary component responsible for absorbing and retaining fluids. The absorbent core (4) may be a multi-functional structure designed for efficient fluid management. The absorbent core (4) is typically enclosed within a core wrap (12), which is a layer of material (typically a nonwoven) that surrounds the absorbent material. The absorbent material (13) within the core is generally responsible for absorbing and retaining fluids. This material may be selected from a variety of suitable options, including superabsorbent polymers (SAPs) and cellulose fibers. The absorbent material (13) herein preferably comprises a mixture of superabsorbent particles with intermixed and dispersed cellulose fibers therein, for high absorbent capacity and good fluid distribution properties. The absorbent core (4) may further comprise an acquisition and distribution layer, ADL, (14) positioned between the liquid permeable topsheet (2) and the absorbent core (4). The ADL (14) is generally designed to rapidly acquire discharged bodily fluids, such as urine, and then distribute these fluids to the absorbent core (4) for retention. This may help to prevent pooling and rewetting of the topsheet (2), maintaining a dry and comfortable surface against the wearer's skin. The acquisition layer may typically be less hydrophilic than the absorbent layer and may typically be made from a nonwoven, such as a spunbond nonwoven, a carded nonwoven, a through-air bonded, thermo-pressure bonded, or chemically bonded carded nonwoven. The absorbent core (4) may comprise one or more channels (15) that are substantially free of absorbent material. These channels (15) generally facilitate fluid distribution within the absorbent core (4) and prevent bunching or clumping of the absorbent material when the core is wetted. The channels may be embossed but are preferably formed by bonding top and bottom layers of the core wrap together at attachment areas substantially corresponding to the channel shape(s). Exemplary suitable channels are illustrated in Fig.6A-C.

To further prevent the risk of side leakage, the absorbent article (1) includes a pair of barrier leg cuffs (5, 5'). The barrier leg cuffs (5, 5') are strategically positioned along the longitudinal edges of the chassis to form a seal against the wearer's legs. As a feature of this disclosure, the topsheet (2) extends and forms at least a portion of these barrier leg cuffs (5, 5'). This eliminates the need for additional materials and bonding steps, potentially reducing leak paths and improving manufacturing efficiency.

In addition to the components described above, the absorbent article (1) may incorporate one or more further features designed to enhance its fit, comfort, and functionality. For absorbent articles (1) configured as open diapers, elastic ears may be included at the rear corners of the chassis. These elastic ears provide improved fit and conformability around the wearer's waist and contribute to a more secure and comfortable fit. A fastening system may also be included with open diaper configurations, which enables the securement of the absorbent article (1) around the wearer's body. The fastening system may comprise adhesive tapes, hook-and-loop fasteners, or other suitable mechanical closures. In embodiments utilizing hook-and-loop fasteners, a landing zone or receiving area may be provided on the outer surface of the front portion of the absorbent article (1) to facilitate secure and adjustable fastening. For absorbent articles (1) configured as pull-on pants, elastic front and back waist belts are incorporated into the design. These elastic waist belts are seamed together at the side edges of the absorbent article (1), forming a closed, pant-like configuration. The elastic waist belts provide a snug and comfortable fit around the wearer's waist, allowing the absorbent article (1) to be easily pulled up and down like regular underwear.

### THE BARRIER LEG CUFFS

A key aspect of the barrier leg cuffs (5, 5') herein is that the topsheet (2) forms at least a portion of each of the barrier leg cuffs (5, 5'). This may be achieved by extending the topsheet material beyond the longitudinal edges of the absorbent core (4) and configuring it to form at least a portion (preferably the majority, even more preferably substantially all of) the inner facing layer (5t, 5t') of the cuffs. This single-web construction streamlines production by reducing component count and bonding steps.

Preferably, the cuffs (5, 5') herein are multi-layer cuffs meaning that each cuff is made out of at least two layers of material, more preferably said material being a hydrophobic nonwoven that may be selected from the nonwovens described herein.

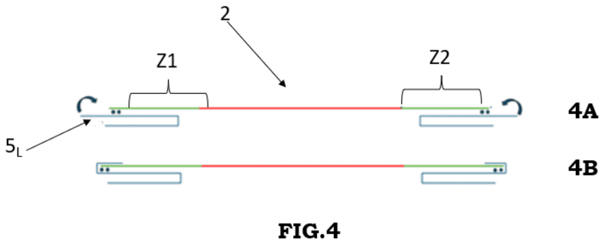

The hydrophobic lateral regions (Z1, Z2) of the topsheet (2) may be joined to a further distinct outer facing layer (5_{L}, 5_{L}') of the barrier leg cuffs (5, 5') with one or more elastic elements being sandwiched therebetween (exemplary forming steps are illustrated in Fig.4A and B). The outer facing layer generally provides additional containment and helps to maintain the overall shape and structure of the barrier leg cuff (5, 5'). The selection of a material for the distinct outer facing layer that differs from that of the topsheet (2) may be advantageous in achieving specific performance characteristics. For instance, the outer facing layer may have a higher basis weight than the topsheet to provide increased structural support and resistance to tearing. This can also optimize the overall cost of the absorbent article, as a higher basis weight material is only used in the region where increased strength and durability are needed, rather than across the entire surface of the topsheet. This also contributes to reducing material usage, which may be advantageous from an environmental point of view. Alternatively, a lower basis weight outer facing layer may be selected to enhance flexibility. For example, the topsheet may have a basis weight of 15 gsm, while the outer facing layer has a basis weight of 20 gsm or vice versa. A higher bulk density outer facing layer may provide improved fluid containment, while a lower bulk density outer facing layer may enhance breathability. The outer facing layer may also have a smaller fiber diameter than the topsheet to create a tighter pore structure and improve its barrier properties. These examples are merely illustrative, and the selection of the outer facing layer's properties will depend on the desired balance of performance characteristics for the specific absorbent article design.

Alternatively, the hydrophobic region (Z1, Z2) of the topsheet (2) may comprise an inner folded edge with one or more elastics encapsulated therein to form each of the pair of barrier leg cuffs (5, 5'), and the cuffs may be free of a further distinct layer.

In embodiments with a distinct outer facing layer, the material may be the same as or different from that of the topsheet (2). Suitable materials include nonwoven, film, or combinations thereof. **In** one embodiment, the distinct outer facing layer is part of the liquid impermeable backsheet (3) of the absorbent article (1). The distinct outer facing layer is preferably part of the outer cover (10) of the absorbent article (1). The outer cover (10) may be sized to be larger than the backsheet (3), such that it extends beyond the backsheet (3) to even exclusively form the distinct outer facing layer.

Each barrier leg cuff, as described herein, may comprise both an inner cuff element (as described herein) and an outer cuff element, wherein the outer cuff element comprises an outer folded edge and from 1 to 3 outer elastic elements, wherein the outer elastic elements are disposed proximate the outer folded edge; and wherein the outer elastic elements are encapsulated by the outer cuff element within an outer fold thereof. This provides enhanced softness and prevents direct contact of the elastic elements with the skin. Preferably, the outer cuff element of each of the pair of barrier leg cuffs is positioned at a lateral edge of said article, said lateral edge extending substantially parallel to a longitudinal axis and preferably wherein a pair of said lateral edges together with a pair of oppositely disposed transversal edges extending substantially perpendicular to the longitudinal axis form a perimeter of a chassis of said article. This provides a neat and defined edge to the absorbent article.

According to a preferred embodiment of the disclosure, the outer cuff element at least partly overlaps the backsheet (3) on the skin-facing side of the backsheet (3), and preferably wherein at least a portion of the outer cuff element is disposed directly or indirectly, preferably directly, over the backsheet (3).

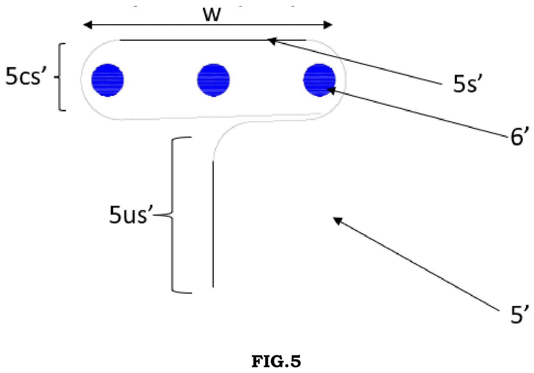

In some embodiments, as for example shown in Fig.5, the barrier leg cuffs (5, 5') comprise a cutaneous sealing strut (5cs, 5cs') and an upstanding connecting strut (5us, 5us') adjoining said cutaneous sealing strut. The configuration of the cutaneous sealing strut and upstanding connecting strut can significantly influence the performance and comfort of the cuffs. A specific and beneficial configuration is a substantially T-shaped cuff, preferably when viewed in a cross-section taken about an axis substantially perpendicular to the longitudinal axis (y). This T-shape may provide a superior combination of effective fluid containment and gentle contact with the skin. The T-shape allows the cuff to stand up effectively, while providing a wider, softer contact area against the skin, minimizing pressure points and enhancing wearer comfort. The cutaneous sealing strut may be substantially symmetrical about an axis following a longest dimension of the upstanding connecting strut, typically with said longest dimension being substantially perpendicular to the width (w) of the skin-contact surface. Also, it is preferable that the same number of inner elastic elements are positioned on either side of the upstanding connecting strut.

The cutaneous sealing strut may have a first longest dimension, and the upstanding connecting strut may have a second longest dimension; and a ratio of the first longest dimension to the second longest dimension may be from 0.8 to 2.5, preferably from 0.9 to 2.2, even more preferably between 1 and 2. This dimensional relationship, when optimized, may provide a superior balance between the cuff's sealing performance and wearer comfort.

### METHOD OF MANUFACTURE

The present disclosure also relates to a method of manufacturing the absorbent article (1) herein described. The method involves several steps comprising: providing the constituent layers, positioning the absorbent core, creating the zone treatment on the topsheet (whether inline or offline by a topsheet manufacturer before use in a conversion line for article production), and forming the barrier leg cuffs (5, 5'). The method allows for efficient and cost-effective production of an absorbent article with enhanced leakage protection and fluid management.

First, a topsheet (2), a liquid impermeable backsheet (3), and an absorbent core (4) are provided. These components are typically supplied in roll form, ready to be processed in a continuous manufacturing line. The topsheet (2) material may be selected based on its softness, liquid permeability, and ability to be zone treated. The backsheet (3) material may be selected for its liquid impermeability and, optionally, breathability. The absorbent core (4) may be pre-formed or formed inline during the article conversion process, and typically comprising a mixture of superabsorbent polymers (SAPs) and cellulose fibers enclosed within a core wrap (12).

Next, the absorbent core (4) is positioned at least partially between the topsheet (2) and the backsheet (3). This positioning can be achieved using automated placement equipment that accurately dispenses and aligns the absorbent core (4) onto the backsheet (3) or the topsheet (2), depending on the specific manufacturing setup. Adhesive may be applied to one or both of these layers to secure the absorbent core (4) in place.

The topsheet (2) preferably undergoes a zone treatment either before it is spooled into a reel by a topsheet supplier or done directly inline during the article conversion process. This creates a central hydrophilic section and at least two hydrophobic lateral regions (Z1, Z2) oppositely disposed relative to a longitudinal centerline of the absorbent article (1). The zone treatment may be achieved through various methods, including selective application of a hydrophilic spin finish, application of a hydrophobic coating, or construction of the lateral regions from hydrophobic fibers. If a hydrophilic spin finish is used, it is typically applied to the central section of the topsheet (2) using spraying, printing, or other suitable methods, ensuring a controlled and uniform distribution. If a hydrophobic coating is used, it is applied to the lateral regions (Z1, Z2) using similar techniques.

The zone-treated topsheet (2) may be extended beyond the longitudinal edges of the absorbent core (4). These extended portions of the topsheet (2) are then generally folded to form at least an inner facing layer of a pair of barrier leg cuffs (5, 5'). The folding process may involve the use of folding boards, rollers, or other mechanical devices to create a precise and consistent fold. The folded edges may then be secured using adhesive bonding, thermal bonding, or other suitable methods.

In some embodiments, the hydrophobic lateral regions (Z1, Z2) of the topsheet (2) are joined to a further distinct outer facing layer (5L, 5L'). The outer facing layer provides additional containment and may be made of a nonwoven, film, or combinations thereof. The securing of the topsheet (2) to the outer facing layer can be achieved using adhesive bonding, thermal bonding, ultrasonic bonding, or other suitable methods. One or more elastic elements may be sandwiched between the topsheet (2) and the outer facing layer (5L, 5L') to provide elasticity to the cuffs.

In an embodiment, as shown for example in Fig.4, the outer facing layer(s) (5L, 5L') are joined to the hydrophobic lateral regions (Z1, Z2) of the topsheet (2) before the folding step of the extended portions of the topsheet (2) as described above. As mentioned herein, one or more elastics may be sandwiched between each of the hydrophobic lateral regions (Z1, Z2) and the outer facing layer (5L, 5L'). A portion of the outer facing layer(s) (5L, 5L') may be folded over the hydrophobic lateral regions (Z1, Z2) such to enclose the elastics therein.

This method of manufacturing allows for the creation of an absorbent article with improved leakage prevention and fluid management, using a simplified construction that reduces material costs and streamlines the manufacturing process.

It is assumed that the present disclosure is not restricted to any form of embodiment described above and that some modifications can be made to the presented embodiments of the invention without departing from the scope of the embodiments as set forth in the claims that follow.

## Claims

1. An absorbent article (1) comprising:
a topsheet (2);
a liquid impermeable backsheet (3);
an absorbent core (4) disposed at least partially between the topsheet (2) and the backsheet (3); and
a pair of barrier leg cuffs (5, 5'), each barrier leg cuff (5.5') comprising an inner cuff element (5a, 5a');
and **characterised in that** the topsheet (2) forms at least a portion of each of said barrier leg cuffs (5,5'); and **in that** the topsheet (2) is zone treated so that a central section (Cs) of the topsheet (2) is hydrophilic and is interposed between at least two hydrophobic lateral regions (Z1, Z2) that are oppositely disposed relative to a longitudinal centerline of the absorbent article; and preferably wherein the hydrophobic lateral regions (Z1, Z2) of the topsheet (2) form at least a portion of an inner facing layer (5t, 5t') of the barrier leg cuffs (5, 5').

2. An absorbent article (1) according to claim 1, wherein the hydrophobic region (Z1, Z2) of the topsheet (2) is joined to a further distinct outer facing layer (5_{L}, 5_{L}') of the barrier leg cuffs (5, 5') with one or more elastic elements (6, 6') being sandwiched therebetween.

3. An absorbent article (1) according to any of the preceding claims, wherein the hydrophobic region (Z1, Z2) of the topsheet (2) comprises an inner folded edge (5af, 5af') with one or more elastic elements (6, 6') encapsulated therein to form each of the pair of barrier leg cuffs (5, 5'), and preferably wherein said cuffs are free of a further distinct layer.

4. An absorbent article (1) according to any of the preceding claims, wherein the topsheet (2) is a hydrophobic nonwoven that is treated with a hydrophilic spin finish within said central section such that the hydrophobic lateral regions (Z1, Z2) are substantially free of said hydrophilic spin finish.

5. An absorbent article (1) according to any of the preceding claims, wherein the topsheet (2) is a nonwoven layer selected from the group consisting of: spunbond, meltblown, carded, and combinations thereof.

6. An absorbent article (1) according to any of the preceding claims, wherein each barrier leg cuffs (5, 5') is an upstanding barrier leg cuff and comprise a single layer of nonwoven web.

7. An absorbent article (1) according to any of claims 1-5, wherein each barrier leg cuffs (5, 5') is an upstanding barrier leg cuff and comprise at least two layers of nonwoven web, and preferably wherein the majority of said cuff is free of adhesive or other joining means between said two layers so that an air gap may be formed between them.

8. An absorbent article (1) according to any of claims 2-7, wherein the distinct outer facing layer (5_{L}, 5_{L}') is selected from a material that is the same or different to that of the topsheet (2), and preferably wherein said material is selected from the group consisting of nonwoven, film or combinations thereof.

9. An absorbent article (1) according to claim 8, wherein the material of the distinct outer facing layer (5_{L}, 5_{L}') is a nonwoven selected from the group consisting of spunbond, meltblown, and combinations thereof.

10. An absorbent article (1) according to any of the preceding claims, further comprising an outer cover (11) that is associated with the backsheet (3), preferably at a garment facing surface thereof; more preferably wherein the outer cover (11) is a hydrophobic nonwoven layer.

11. An absorbent article (1) according to any of claims 2-10, wherein the distinct outer facing layer (5_{L}, 5_{L}') is part of the liquid impermeable backsheet (3) of the absorbent article (1); and preferably wherein the backsheet (3) is a film.

12. An absorbent article (1) according to any of claims 10-11, wherein the distinct outer facing layer (5_{L}, 5_{L}') is part of the outer cover (11) of the absorbent article (1).

13. An absorbent article (1) according to claim 12, wherein the outer cover (11) is sized to be larger than the backsheet (3), preferably such that it may extend beyond the backsheet (3) to exclusively form the distinct outer facing layer (5_{L}, 5_{L}').

14. An absorbent article (1) according to any of the preceding claims, wherein each of the cuffs (5, 5') comprise a cutaneous sealing strut (5cs, 5cs') and an upstanding connecting strut (5us, 5us') adjoining said cutaneous sealing strut (5cs, 5cs'), preferably wherein each of the cuffs (5, 5') is substantially T-shaped.

15. An absorbent article (1) according to claim 14, wherein the cutaneous sealing strut (5cs, 5cs') has a first longest dimension, and the upstanding connecting strut (5us, 5us') has a second longest dimension; and wherein a ratio of first longest dimension/second longest dimension is from 0.8 to 2.5, preferably from 0.9 to 2.2, even more preferably between 1 and 2; preferably wherein the first longest dimension runs parallel to a width (w) of a skin-contact surface (5s, 5s') and the second longest dimension runs substantially perpendicular thereto.

16. A method for the manufacture of an absorbent article (1), said article preferably according to any of the preceding claims, comprising the steps of:
providing a topsheet (2);
zone treating the topsheet (2) to form a central hydrophilic section (Zc) and at least two hydrophobic lateral regions (Z1, Z2) oppositely disposed relative to a longitudinal centerline of the absorbent article (1);
providing a liquid impermeable backsheet (3), and an absorbent core (4);
positioning the absorbent core (4) at least partially between the topsheet (2) and the backsheet (3);
extending the topsheet (2) beyond longitudinal edges of the absorbent core (4);
folding the extended portions of the topsheet (2) comprising the hydrophobic lateral regions (Z1, Z2) to form at least an inner facing layer of a pair of barrier leg cuffs (5, 5'); and
optionally securing the hydrophobic lateral regions (Z1, Z2) of the topsheet (2) to a further distinct outer facing layer (5L, 5L') to form a pair of barrier leg cuffs (5, 5').
